# EUROPEAN PATENT APPLICATION

(11) **EP 4 748 921 A1**
(43) Date of publication of application: **27.05.2026**
(21) Application number: 24383275.5
(22) Date of filing: 26.11.2024
(51) Int. Cl.: C12N 1/20, A23L 33/135, A61K 35/744

(54) **LACTOCOCCUS LACTIS SUBSP. LACTIS STRAIN AND ITS USES**

(71) Applicant: Nea Thea 7, S.L., 37892 Sieteiglesias de Tormes (Salamanca) (ES)
(72) Inventor: García Martín, Elsa, 37892 Sieteiglesias de Tormes (ES); De la Cruz Sánchez, Rebeca, 37892 Sieteiglesias de Tormes (ES); Martín Diana, Ana Belén, 47009 Valladolid (ES); Rico Bargues, Daniel, 47002 Valladolid (ES); Requena Rolania, Teresa, 28006 Madrid (ES); Olmo López, Rocío, 37008 Salamanca (ES); Martín Quijada, Narciso, 37008 Salamanca (ES); Monte Vázquez, Enrique, 37008 Salamanca (ES)
(74) Representative: Pons IP

(57) **Abstract**

The present invention relates to the strain *Lactococcus lactis* subsp. *lactis* CECT 31096, to its cellular components, metabolites, and secreted molecules, and to compositions comprising the above products, as well as to their uses as a medicament, preferably for the prevention and/or reduction of hypertension, or as a food composition or nutraceutical.

## Description

The current invention belongs to the technical field of food microbiology, particularly of probiotic products. The current invention refers to the strain of *Lactococcus lactis* subsp. *lactis* CECT 31096, isolated from raw donkey milk, to its cellular components, metabolites, and molecules produced during the fermentation process of the milk, to the compositions which comprise the mentioned strain or any of its components, as well as to its uses as a medicine or as food compositions or nutraceuticals.

### STATE OF THE ART

Donkey milk and its beneficial properties have been known for thousands of years. In Spain, donkey milk had its peak during the first half of the 20th century, when the donkey was used for domestic chores and its milk was used as a natural remedy for some illnesses such as colds, coughs, pneumonia and tuberculosis, among others. However, the technological development of the agricultural world and the use of more productive species in the dairy industry led to a decline in the utilisation of this animal. In recent years, scientific interest in donkey milk has increased significantly due to its nutritional and functional elements (Vincenzetti et al., 2017, Role of Proteins and of Some Bioactive Peptides on the Nutritional Quality of Donkey Milk and Their Impact on Human Health. Beverages, 3(4), 34*).*

In terms of its composition, it is the milk that has the greatest similarity to human milk, since it has a low protein content (1.5-1.8%), a similar lipid profile and a high lactose concentration. Donkey milk has a low fat content and its lipid profile is remarkable for its high content of polyunsaturated fatty acids, mainly of the omega-3 family (Gastaldi et al., 2010. Donkey's milk detailed lipid composition. Frontiers in bioscience, 2, 537-546). Furthermore, one of the most outstanding nutritional characteristics is its content in fat-soluble vitamins (vitamins A and D) and in vitamins B9, B12 and selenium *(*Vincenzetti et al., 2021. Vitamins in Human and Donkey Milk: Functional and Nutritional Role. Nutrients, 13(5), 1509*).* On the other hand, the proportion of calcium and phosphorus it contains and the high lactose content, favours the calcium absorption in the body. This means that, although donkey milk contains a lower level of calcium than other milks, a greater amount of this essential mineral is absorbed *(*Derdak et al., 2020, Insights on Health and Food Applications of Equus asinus (Donkey) Milk Bioactive Proteins and Peptides-An Overview. Foods, 9(9), 1302*;* Aspri et al., 2016, Donkey milk: An overview on functionality, technology, and future prospects. Food Reviews International, 33(3), 316-333*).*

Besides the important nutritional value of donkey milk, it is of interest its bioactive and healthy properties that also reside in polyphenolic compounds of natural origin derived from the animal's nourishment and that provide the capacity to neutralize certain free radicals. Therefore, they exhibit both antioxidant capacity and reducing power. Moreover, it is important to highlight that the inherent nutritional and bioactive properties of milk can be enhanced after the fermentation process, being the protein fraction the responsible one. This is due to the fact that during the fermentation process it produces peptide fragments with high bioactive value, mainly antioxidant and antihypertensive capacity (Cavalcanti et al, 2021, Donkey milk and fermented donkey milk: are there differences in the nutritional value and physicochemical characteristics? LWT - FoodScience and Technology 144, 111239*).*

Different scientific studies developed with clinical trials have shown that donkey milk, due to its low saturated fat content, has health benefits such as cholesterol reduction through LDL reduction *(*Aspri et al., 2016, Donkey milk: An overview on functionality, technology, and future prospects. Food Reviews International, 33(3), 316-333; Magrone, & Jirillo, 2014, Disorders of Innate Immunity in Human ageing and effects of Nutraceutical Administration. Endocrine, Metabolic & Immune Disorders. Drug Targets, 14(4), 272-282*)*. Studies have also shown that donkey milk can be an alternative to conventional milks in people with cow's milk protein allergy or intolerance (CMPA). This allergic reaction develops mostly in childhood, mainly in newborns who are not breastfed and receive infant formula and later cow's milk. The allergy is developed as a reaction to an alpha-casein, which is present in high quantities in cow's milk, but is not found in human or donkey's milk. In addition, the structure of caseins found in donkey milk is very similar to the structure of those found in human breast milk, which reduces the likelihood of developing allergies to other caseins and makes it much more digestible, up to 70%. (Sarti et al., 2019, Donkey's Milk in the Management of Children with Cow's Milk protein allergy: nutritional and hygienic aspects. The Italian Journal of Pediatrics, 45(1*);* Derdak et al., 2020, Insights on Health and Food Applications of Equus asinus (Donkey) Milk Bioactive Proteins and Peptides-An Overview. Foods, 9(9), 1302*)).*

Other recent clinical trials have demonstrated the immunomodulatory activity of donkey milk in the maintenance of the natural microbiota of the human intestine. The balance of the intestinal immune system and the decrease of inflammatory states are due to an adequate intestinal microbiota, which improves different metabolic pathways such as the processing of lipids or glucose.

For all these reasons, donkey milk is proposed as a nutraceutical in human nutrition and possibly also in animal nutrition, not only for infants but also for people suffering from osteogenesis, gastrointestinal diseases, hypercholesterolemia, and even for healthy individuals who want to follow a healthy diet and provide a quality product on a daily basis *(*Sarti et al., 2019, Donkey's Milk in the Management of Children with Cow's Milk protein allergy: nutritional and hygienic aspects. Italian Journal of Pediatrics, 45, 102*;* Losinno, & Bragulat, 2019, Leche de burra en cases de alergia a la leche de vaca en humanos. Un desafio para Argentina. Ab Intus, 3*).*

Furthermore, it is important to highlight that immunoglobulins, lysozymes and lactoferrins contained in milk jointly contribute to microbial growth in the digestive tract and thus help to reduce gastrointestinal infections.

Milk consumption requires a milk sanitization in order to guarantee the safety of the product. Pasteurization offers an advantage over commercial hygienisation systems such as ultra-pasteurization or ultra-high temperature processing (UHT) because it maintains the milk at a lower temperature, allowing that the nutritional and bioactive compounds remain active during its storage, especially those with a thermolabile nature. Pasteurization also does not modify the sensory properties as significantly as commercial heat treatments do.

On the other hand, the fermentation process is conventionally used to produce milk with improved nutritional properties associated with the probiotic activity of the bacteria that carry out these processes and with the production of compounds with greater digestibility and bioaccessibility. It is also important to note the reduction in lactose, which favours its ingestion by lactose intolerant people, as well as improving the preservation of the milk itself associated with a pH reduction (from 7.2 to 4.6) and an increase in lactic acid concentration.

In addition, donkey milk contains complex oligosaccharides, which are able to pass through the digestive tract and reach the large intestine, where they are used by the microorganisms that are present there. This is the reason why donkey milk is considered to have a prebiotic effect which, in combination with probiotic bacteria, can generate a symbiotic effect on the gastrointestinal system.

Probiotic bacteria (probiotics) have been defined by the FAO (Food and Agriculture Organization) as living/viable microorganisms that, when administered in appropriate doses, are able to provide health benefits to the host. Among other effects, they facilitate the digestion and absorption of nutrients (including lactose), they balance the gut microbiota and strengthen the immune system, thus protecting the individual against infections and chronic inflammatory conditions. Furthermore, in order for a product composed of microorganisms to be considered as a probiotic, there must be scientific evidence showing that its consumption has a beneficial effect on the host's health.

Therefore, the identification of new lactic acid bacteria with probiotic potential and the development of a dairy product with these bacteria, would mean obtaining a complete and functional food, as it would be composed of a combination of probiotic microorganisms and prebiotic compounds that would serve as their food.

### DESCRIPTION OF THE INVENTION

The present invention concerns a new lactic acid bacterium, taxonomically classified as *Lactococcus lactis* subsp. *lactis* and with accession number CECT 31096. The *Lactococcus lactis* subsp. *lactis* strain with accession number CECT 31096 was isolated from raw donkey milk, and it was deposited on 9^{th} July 2024 under the Budapest Treaty in the Spanish Type Culture Collection as International Depositary Authority (Edificio 3 CUE, Parc Científic Universitat de Valencia, Catedrático Agustin Escardino, 9, 46980, Paterna (Valencia) SPAIN). It is also intended to extend the protection of the patented invention to all those compositions comprising the strain with accession number CECT 31096, particularly food or nutraceutical compositions such as products made from donkey milk and fermented products (with that strain or in combination with other bacteria).

Another aspect of the present invention is related to a strain derived from the strain *Lactococcus lactis* subsp. *lactis* CECT 31096 wherein said strain maintains or improves the capabilities of the original strain, described throughout the present invention. The derived microorganism can be produced either naturally (e.g. spontaneous mutants) or intentionally, by methods of mutagenesis known in the state of the art such as, but not limited to, growth of the original microorganism in the presence of mutagenic or stress-causing agents, or by genetic engineering directed at modifying specific genes. Thus, in a preferred realization, the strain of the invention or the strain derived from the species *L. lactis* CECT 31096 is a genetically modified mutant or a spontaneous mutant. The terms mutant strain or derived strain may be used interchangeably.

The *L. lactis* CECT 31096 strain or any mutant or derived from it can be used in any way that exerts the described effects. According to a preferred realization of the present invention, the *L. lactis* CECT 31096 strain is in the form of viable cells (cultivable or non-cultivable), or according to another preferred embodiment of the invention the *L. lactis* CECT 31096 strain is in the form of non-viable cells (`dead' cells inactivated by any known technique such as, but not limited to, heat, freezing or ultraviolet radiation). Thus, in a more preferred embodiment, the non-viable cells of strain *L. lactis* CECT 31096 are selected from the list consisting of: heat-treated, frozen, freeze-dried or treated by ultraviolet radiation. As used here, the term 'non-viable' (or 'inanimate', 'inactive' or 'inactivated', terms which may be used interchangeably herein), refers to any microorganism that is metabolically or physiologically inactive, that is to say, that does not maintain its metabolic activity and elongation capacity after administration of nutrients. Viability is independent of the ability of the microorganism to form colonies on solid media.

Another aspect of the present invention relates to the cellular components, metabolites, secreted molecules or any combination thereof, obtained from the strain of the invention, derived strain, or from a combination of microorganisms comprising at least one strain of the invention and/or derived strain.

Bacterial cellular components could include cell wall components (such as, but not limited to, peptidoglycan), nucleic acids, membrane components, or other components such as proteins, lipids and carbohydrates and combinations thereof, such as lipoproteins, glycolipids or glycoproteins. Metabolites include any molecule produced or modified by the bacterium as a result of its metabolic activity during growth, its use in technological processes (e.g. food or drug manufacturing processes), during the product storage or during the gastrointestinal transit. Examples of such metabolites are, but are not limited to, organic and inorganic acids, proteins, peptides, amino acids, enzymes, lipids, carbohydrates, lipoproteins, glycolipids, glycoproteins, vitamins, salts, metals or nucleic acids. Secreted molecules include any molecule exported or released to the exterior by the bacteria during growth, its use in technological processes (e.g. food or drug manufacturing), product storage or gastrointestinal transit. Examples of such molecules are, but are not limited to, organic and inorganic acids, proteins, peptides, amino acids, enzymes, lipids, carbohydrates, lipoproteins, glycolipids, glycoproteins, vitamins, salts, metals or nucleic acids.

Another aspect of the present invention relates to a composition, hereinafter 'composition of the invention', comprising the strain of the invention, derived strain and/or the cellular components, metabolites, secreted molecules of the strain of the invention or derived strain, or any combination thereof.

The composition, defined broadly, is a set of components consisting of at least the strain of the invention and/or the derived strain at any concentration; or at least the cellular components, metabolites, secreted molecules of the strain of the invention or the derived strain, or any combination thereof; or a combination thereof.

In a preferred embodiment, the composition of the invention has a concentration of the strain of the invention, and/or the derived strain, of between 10⁴ and 10¹⁴ colony forming units (cfu) per gram or milliliter of final composition, preferably 10⁸ CFU/mL.

In another preferred embodiment, the composition of the invention may further comprise at least one additional microorganism, wherein said microorganism may belong to the same or different species of the strain of the invention or derived, and/or their cellular components, metabolites or secreted molecules, or any combination thereof. For example, but not limited to, the additional microorganism that may be part of such composition is at least one lactic acid bacterium or bifidobacterium of intestinal, food or environmental origin. The lactic bacterium is selected from the list comprising, but not limited to, a bacterium of the genus *Bifidobacterium, Lactobacillus, Lactococcus, Enterococcus, Propionibacterium, Leuconostoc, Weissella, Pediococcus* and *Streptococcus.* Most preferably, the additional microorganism is *Streptococcus thermophilus* and/or *Lactobacillus delbrueckii* subsp. *bulgaricus.*

In a preferred embodiment, alone or in combination with other embodiments, the composition of the invention is a dairy product, preferably a fermented milk product. Preferably, the milk product comprises milk isolated from a female individual that belongs to the species *Equus africanus* subsp. *asinus,* more preferably donkey milk.

In a more preferred embodiment, the dairy product is donkey milk, preferably fermented donkey milk.

In a preferred embodiment, alone or in combination with the above embodiments, the milk product, preferably fermented milk product, more preferably fermented donkey milk, comprising the strain of the invention, derived strain and/or the cellular components, metabolites, secreted molecules of the strain of the invention or derived strain (or any combination thereof), has been previously pasteurized (in this case, before obtaining the fermented milk product).

In another preferred embodiment, alone or in combination with other preferred embodiments, the milk product, preferably the fermented milk product is freeze-dried afterwards.

Furthermore, the composition of the invention can be formulated for pharmaceutical administration, that is to say, forming part of pharmaceutical products to be administered to the subject by any means of administration. Thus, in a particular embodiment, the composition of the invention is a pharmaceutical composition, hereinafter also referred to as 'pharmaceutical composition of the invention'. The pharmaceutical composition is a set of components consisting of at least the strain of the invention and/or the derived strain in any concentration; or at least the cellular components, metabolites, secreted molecules of the strain of the invention or derived strain, or any combination thereof, which has at least one application in improving the physical or physiological or psychological well-being of an individual, involving an improvement of the general state of his health or a reduction of the risk of disease. The said pharmaceutical composition can be a medicament, hereinafter also referred to as the medicament of the invention.

Thus, in another aspect, the invention relates to the strain of the invention, a strain derived therefrom, a cellular component, metabolite, secreted molecule or any combination thereof obtained from the strain of the invention or strain derived therefrom, or the composition of the invention, for its use as a medicament.

The term medicament has a narrower meaning than the 'pharmaceutical composition' as defined in the present invention, since medicament necessarily involves a preventive or therapeutic effect. The medicament referred to in this invention may be for human or veterinary use. The term 'medicament for human use' means any substance or combination of substances that presents itself having properties for treating or preventing diseases in human beings or which may be used in or administered to human beings with the aim of restoring, correcting or modifying physiological functions by exerting a pharmacological, immunological or metabolic action, or of making a medical diagnosis. The term 'veterinary medicament' means any substance or combination of substances presented as having properties for treating or preventing disease in animals or which may be administered to animals with the aim of restoring, correcting or modifying physiological functions by exerting a pharmacological, immunological or metabolic action, or of making a veterinary diagnosis. Premixtures for medicated feedingstuffs' prepared for incorporation into a fodder shall also be considered to be 'veterinary medicaments'.

In a particular embodiment, the pharmaceutical composition of the invention further comprises at least one pharmaceutically acceptable vehicle and/or excipient.

The `vehicle' or carrier is preferably an inert substance. The function of the vehicle is to facilitate the incorporation of other compounds, to allow for better dosage and administration or to give consistency and shape to the pharmaceutical composition. Therefore, the vehicle is a substance which is used in the medicament to dilute any of the components of the pharmaceutical composition of the present invention to a given volume or weight; or which even without diluting these components can allow a better dosage and administration or giving consistency and shape to the medicinal product. When the dosage form is liquid, the pharmaceutically acceptable vehicle is the diluent.

The term 'excipient' refers to a substance that aids the absorption of any of the components of the composition of the present invention, stabilizes those components or aids the preparation of the pharmaceutical composition in the sense of giving it consistency or providing flavours that make it more palatable. Thus, the excipients could have the function of holding the components together such as for example starches, sugars or celluloses, sweetening function, colouring function, function of protecting the medicinal product such as for example to insulate it from air and/or moisture, filling function of a tablet, capsule or any other form of presentation such as for example dibasic calcium phosphate, disintegrating function to facilitate the dissolution of the components and their absorption in the intestine, without excluding other types of excipients not mentioned in this paragraph. The term 'excipient' is therefore defined as matter which, included in the dosage forms, is added to the active substances or their associations to enable their preparation and stability, to modify their organoleptic properties or to determine the physico-chemical properties of the pharmaceutical composition and its bioavailability. The `pharmaceutically acceptable' excipient must allow the activity of the compounds of the pharmaceutical composition, that is to say, be compatible with those compounds.

Furthermore, as understood by the person skilled in the art, the excipient and the vehicle must be pharmacologically acceptable, i.e. the excipient and the vehicle must be permitted and evaluated so as not to cause harm to the organisms to which they are administered.

Alternatively to the pharmaceutical composition, the composition of the invention may also be a nutritional composition, also referred to herein as the 'nutritional composition of the invention'. Thus, in another particular embodiment, the composition of the invention is a nutritional composition.

The term 'nutritional composition' of the present invention refers to that food which, independently of providing nutrients to the subject who takes it, beneficially affects one or more functions of the organism, so as to provide a better state of health and well-being.

In a preferred embodiment, the nutritional composition is a food, a supplement, a nutraceutical, a probiotic, a symbiotic or a postbiotic.

In a more preferred embodiment, the food is selected from the list consisting of dairy product, plant product, meat product, snack, chocolate, beverage or infant food.

Examples of dairy products include, but are not limited to, fermented (for example, but not limited to, yoghurt or cheese) or non-fermented (for example, but not limited to, ice cream, butter, margarine, whey). In a more preferred embodiment, the food is a dairy product selected from the list consisting of yogurt, cheese, ice cream, butter, margarine, whey, and any other fermented or non-fermented milk product. In a particular embodiment of the invention, the food is selected from the list consisting of a dairy product or an infant food, preferably a dairy product from donkey milk. More preferably, the dairy product is a fermented dairy product, even more preferably a fermented dairy product from donkey milk.

Examples of plant products include, but are not limited to, a cereal in any form of presentation, fermented or non-fermented. In another more preferred embodiment, the food is a plant product, wherein said plant product is a fermented or non-fermented cereal.

Examples of a beverage include, but are not limited to, any fruit or vegetable juice or non-fermented milk. In another more preferred embodiment, the food is a beverage, wherein said beverage is a fruit or vegetable juice or non-fermented milk.

The term 'supplement', synonymous with any of the terms 'dietary supplement', 'nutritional supplement'; or 'food supplement' is a 'food ingredient' intended to complement the diet. Examples of supplements include, but are not limited to, vitamins, minerals, botanicals, amino acids, and food components such as enzymes and glandular extracts. They are not presented as a substitute for a conventional food or as a single component of a meal or food diet but as an addition to the diet. Furthermore, the supplement may be presented in single or combined form and may be commercialised in the form of, but not limited to, capsules, pills, tablets and other similar forms, powder sachets, liquid ampoules and drop dispensers, and other similar forms of liquids and powders designed to be taken in a single amount.

The term 'nutraceutical' as used in the present invention refers to substances isolated from a food and used in a dosed form that have a beneficial effect on health.

The term 'probiotic' as used in the present invention refers to all those food compositions that contain a sufficient amount of live microorganisms to promote health benefits to the host.

The term 'symbiotic', as used in the present invention, refers to all those food compositions containing a mixture of prebiotic compounds and probiotic microorganisms. As a rule, the prebiotic component promotes the growth and/or metabolic activity and ultimately the effect of the probiotic with which it is combined, such as, but not limited to, the association of fructo-oligosaccharides or galacto-oligosaccharides with bifidobacteria.

The term 'postbiotic' as used in the present invention refers to substances or metabolites produced by the probiotic microorganisms contained in a given food composition and which have a beneficial effect on the health of the host on a nutritional, metabolic and immune level, such as but not limited to metabolites such as vitamin K, short-chain fatty acids or neurotransmitters such as GABA, serotonin or acetylcholine.

Another aspect relates to a composition, preferably a dairy product, obtained from the fermentation of animal milk (previously pasteurized), by the strain of the invention (hereinafter, the fermented composition obtained from the invention). Preferably the animal milk is milk isolated from a female specimen belonging to *Equus africanus* subsp. *asinus,* more preferably donkey milk.

In a preferred embodiment, alone or in combination with other preferred embodiments, the milk is pasteurized. More preferably, the milk is pasteurized donkey milk (for example, donkey milk previously pasteurized upon fermentation with the strain of the invention).

In a preferred embodiment, alone or in combination with other preferred embodiments, the fermented milk product is freeze-dried.

In a preferred embodiment, alone or in combination with other preferred embodiments, the fermentation comprises the addition of at least one other microorganism than the strain of the invention, preferably wherein said microorganism may belong to the same or different species of the strain of the invention or derived, and/or its cellular components, metabolites or secreted molecules, or any combination thereof. For example, but not limited to, the additional microorganism that may be part of such a composition is at least one lactic acid bacterium or bifidobacterium of intestinal, food or environmental origin. The lactic bacteria is selected from the list comprising, but not limited to, a bacterium of the genus *Bifidobacterium, Lactobacillus, Lactococcus, Enterococcus, Propionibacterium, Leuconostoc, Weissella, Pediococcus and Streptococcus.* More preferably, the additional microorganism is *Streptococcus thermophilus* and/or *Lactobacillus delbrueckii* subsp. *bulgaricus.*

In the present aspect, fermentation comprises inoculation or addition of the strain of the invention to the milk (or milk sample), and preferably, at least one other microorganism different from the strain of the invention, such as those mentioned above.

In a preferred embodiment, alone or in combination with other preferred embodiments, fermentation is carried out at a temperature of between 39°C to 46°C, preferably at 42°C.

In another preferred embodiment, alone or in combination with other preferred embodiments, fermentation is carried out for at least 3 hours.

In another aspect, the present invention relates to the strain of the invention, a strain derived from it, a cellular component, metabolite, secreted molecule or any combination thereof obtained from the strain of the invention, the composition of the invention, including the pharmaceutical composition, the fermented composition obtained from the invention, for its use as a medicament.

In another aspect, the present invention relates to the strain of the invention, a strain derived therefrom, a cellular component, metabolite, secreted molecule or any combination thereof obtained from the strain of the invention, the composition of the invention, including the pharmaceutical composition, or the fermented composition obtained from the invention, for its use as an antihypertensive.

In another aspect, the present invention relates to the strain of the invention, a strain derived therefrom, a cellular component, metabolite, secreted molecule or any combination thereof obtained from the strain of the invention, the composition of the invention, including the pharmaceutical composition, or the fermented composition obtained from the invention, for its use in the prevention and/or treatment of hypertension, or related conditions.

In another aspect, the present invention concerns the strain of the invention, a strain derived therefrom, a cellular component, metabolite, secreted molecule or any combination thereof obtained from the strain of the invention, the composition of the invention, including the pharmaceutical composition, or the fermented composition obtained from the invention, for its use as an antioxidant.

In another aspect, the present invention relates to the strain of the invention, a strain derived thereof, a cellular component, metabolite, secreted molecule or any combination thereof obtained from the strain of the invention, the composition of the invention, including the pharmaceutical composition, or the fermented composition obtained from the invention, for its use in the treatment and/or prevention of cow's milk protein allergy (CMPA).

In the present invention, the term 'treatment' refers to combating the effects caused as a consequence of a disease or pathological condition of interest in a subject (preferably a mammal, and more preferably a human) comprising:
(i) inhibiting the disease or pathological condition, i.e. halting its development;
(ii) alleviating the disease or pathological condition, i.e. causing regression of the disease or pathological condition or its symptoms;
(iii) stabilise the disease or pathological condition.

In the present invention, the term 'prevention' refers to avoiding the occurrence of the disease or pathological condition in a subject (preferably a mammal and more preferably a human), in particular when such subject is predisposed to the pathological condition.

Another aspect of the present invention is the non-therapeutic use of the strain of the invention, a strain derived therefrom, a cellular component, metabolite, secreted molecule or any combination thereof obtained from the strain of the invention, the composition of the invention, or the fermented composition obtained from the invention for the preparation of a food, preferably a dairy food from donkey milk.

Another aspect of the present invention is related to the use of the strain of the invention, or composition of the invention, for fermenting a milk product. Preferably, the milk product is or comprises milk isolated from a female specimen belonging to *Equus africanus* subsp. *asinus.* More preferably, the milk product is, or comprises, donkey milk.

Another aspect of the present invention relates to a method of making a fermented milk product, which comprises carrying out fermentation of previously isolated animal milk (or milk sample), wherein said fermentation comprises inoculating, or adding, the strain of the invention to the milk (or milk sample). Preferably the animal milk is milk isolated from a female individual belonging to *Equus africanus* subsp. *asinus,* more preferably donkey milk.

In a preferred embodiment, alone or in combination with other preferred embodiments, the fermentation also comprises adding at least one other microorganism than the strain of the invention, preferably wherein said microorganism may belong to the same or different species of the strain of the invention or derived, and/or its cellular components, metabolites or secreted molecules, or any combination thereof. For example, but not limited to, the additional microorganism that may be part of such a composition is at least one lactic acid bacterium or bifidobacterium of intestinal, food or environmental origin. The lactic bacteria are selected from the list comprising, but not limited to, a bacterium of the genus *Bifidobacterium, Lactobacillus, Lactococcus, Enterococcus, Propionibacterium, Leuconostoc, Weissella, Pediococcus and Streptococcus.* More preferably, the microorganism different from the strain of the invention is *Streptococcus thermophilus* and/or *Lactobacillus delbrueckii* subsp. *bulgaricus.*

In a preferred embodiment, alone or in combination with other preferred embodiments, the sample of animal milk has been previously pasteurized.

In a preferred embodiment, alone or in combination with other preferred embodiments, fermentation is carried out at a temperature of between 39°C to 46°C, preferably at 42°C.

In another preferred embodiment, alone or in combination with other preferred embodiments, fermentation is carried out for at least 3 hours.

### DESCRIPTION OF THE FIGURES

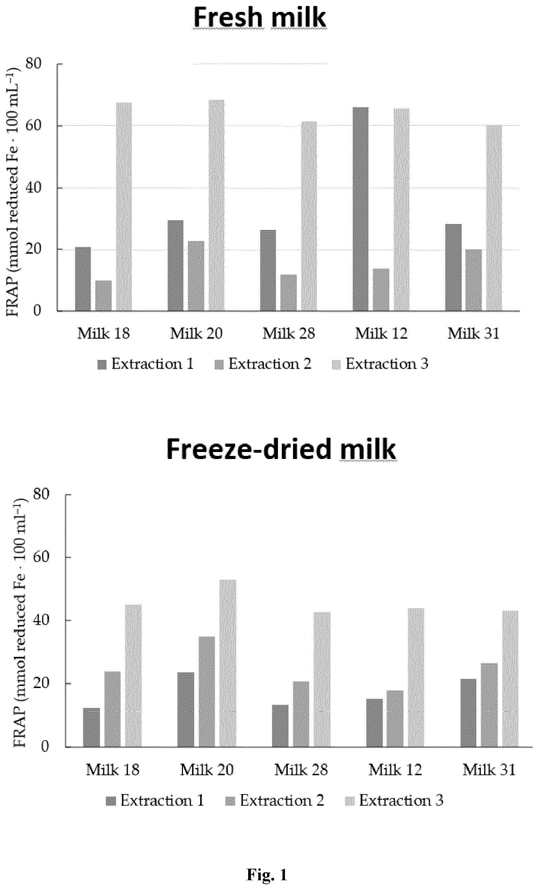

**Figure 1****.** Results of the analysis of the antioxidant properties of donkey milk samples fermented individually with 5 strains of bacteria (identified as 18, 20, 28, 12 and 31) in two forms: fresh and freeze-dried, using the FRAP marker. The graph shows each of the fractions of donkey milk analysed represented as 3 extractions: the first (extraction 1) corresponds to the total matrix, the second (extraction 2) to the peptide fraction and the third (extraction 3) to the phenols.

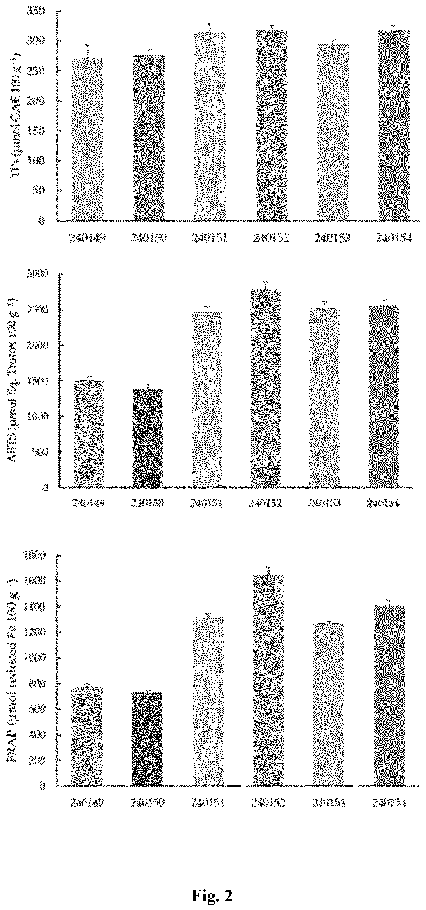

**Figure 2****.** Results of the analysis of the antioxidant properties (evaluation by FRAP, ABTS and TPs markers) of 3 types of donkey milk samples: pasteurised donkey milk (240149, 240150), donkey milk fermented with CHR HANSEN's LAB YF-L903 culture (240151, 240152) and donkey milk fermented with *Lactococcus lactis* subsp. *lactis* CECT 31096 in combination with CHR HANSEN LAB culture YF-L903 (240153, 240154).

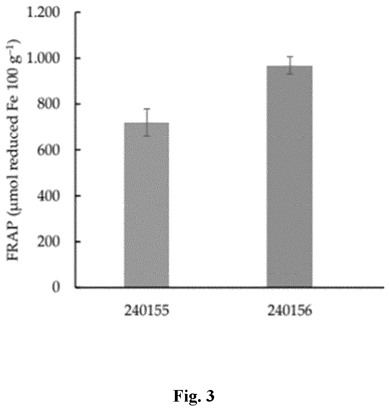

**Figure 3**. Results of the analysis of the antioxidant properties (evaluation by FRAP, ABTS and TPs markers) of 2 fermented donkey milk product samples after digestion: milk fermented only with CHR HANSEN LAB YF-L903 culture (240155) and milk fermented with *Lactococcus lactis* subsp. *lactis* CECT 31096 in combination with CHR HANSEN LAB YF-L903 culture (240156).

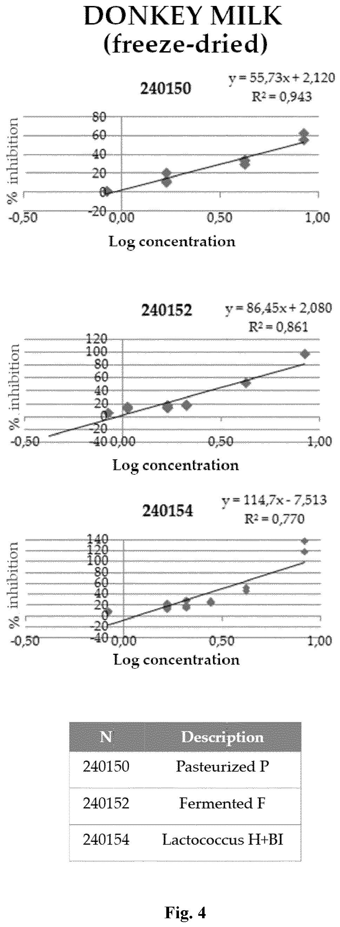

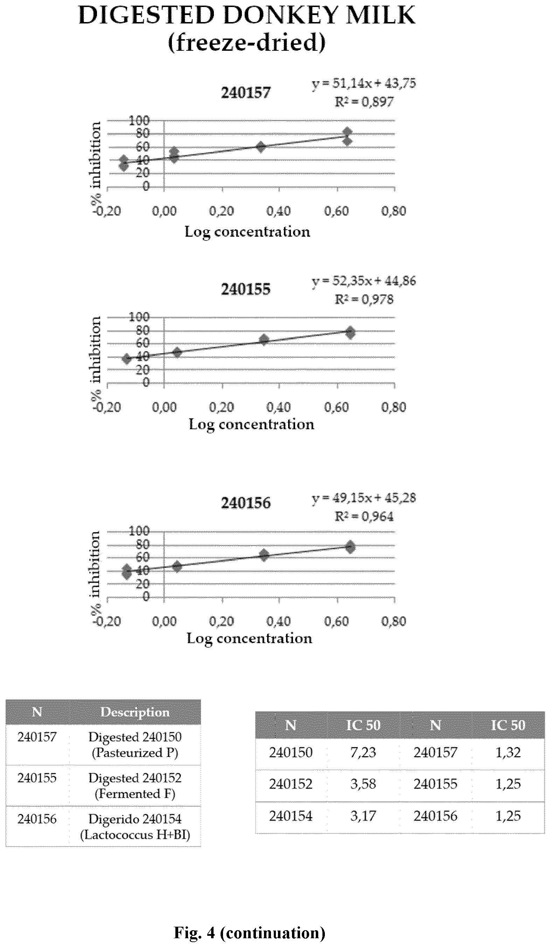

**Figure 4**. Results of the analysis of the antihypertensive properties (evaluation by angiotensin-converting enzyme (ACE) inhibition capacity) of 3 freeze-dried donkey milk samples: freeze-dried pasteurized milk (240150), freeze-dried milk fermented with CHR HANSEN's LAB culture YF-L903 (240152) and freeze-dried milk fermented with *Lactococcus lactis* subsp. *lactis* CECT 31096 in combination with LAB culture YF-L903 from CHR HANSEN (240154) as well as 3 samples of freeze-dried digests of donkey milk carried out by means of a gastrointestinal simulator: freeze-dried digest of pasteurized milk (240157), freeze-dried digest of fermented milk with the LAB culture of YF-L903 from CHR HANSEN (240155) and freeze-dried digest of fermented milk with *Lactococcus lactis* subsp. *lactis* CECT 31096 in combination with CHR HANSEN's LAB culture YF-L903 (240156).

### EXAMPLES

The strain *Lactococcus lactis* subsp. *lactis,* with deposit number CECT 31096, was isolated from fermented raw donkey milk of the Zamorano-Leonesa breed, maintaining the milk for 23 to 28 hours at a temperature of 37°C until the pH decreased to 4.6. Subsequently, the fermented milk was homogenized and serial 1:10 dilutions of the milk were made in a maximum recovery diluent (MRD). Subsequently, 0.100 mL of the 10-5, 10-6, 10-7 and 10-8 dilutions were seeded with digralsky loop in two selective media M17 and MRS (Man, Rogosa and Sharpe solid culture medium) and the plates were incubated under different conditions according to the type of bacteria:
- M17: Incubation at 37°C for 24-48 hours under aerobic conditions and incubation at 37°C for 24-48 hours under anaerobic conditions, that is to say, in hermetic boxes with anaerobic envelope.
- MRS Agar: Incubation at 37°C for 24-48 hours under anaerobic conditions and incubation at 42°C for 24-48 hours under anaerobic conditions and, MRS with pH 5.2, incubation at 37°C for 24-48 hours under anaerobic conditions.

Once the incubations were completed, individual isolates were selected and subcultured in their appropriate medium (M17 or MRS) by streaking and under specific conditions, with the aim of obtaining a pure culture. Based on the colonies obtained, they were subcultured:
- M17 plates, which were incubated at 37°C for 24-48 hours under both aerobic and anaerobic conditions.
- MRS plates, which were incubated at 37°C for 24-48 hours under anaerobic conditions.

From this subculture, the step was repeated 2 more times to purify the isolate in its entirety.

The selected lactic acid bacteria were identified by partial sequencing of the 16S rRNA gene. For this purpose, a liquid culture was started in MRS medium at 37°C in order to increase the density of bacterial cells. The sample was centrifuged and, after discarding the supernatant, DNA was extracted with the commercial NucleoSpin Microbial DNA Mini kit for DNA from microorganisms (Macherey-Nagel, Germany) according to the manufacturer's instructions. In addition, the DNA concentration of the samples was measured with a NanoDrop^{™} microvolume spectrophotometer.

Once the DNA was extracted, the 16S rRNA gene was amplified by PCR using the universal primer pair: 27F (5'- AGAGTTTGATCCTGGCTCAG -3', SEQ ID NO: 1) and 1492R (5'-GGTTACCTTGTTACGACTT-3', SEQ ID NO: 2). Amplification was verified by electrophoresis on a 1% agarose gel. Subsequently, the PCR product obtained was purified using the NucleoSpin Gel and PCR clean-up kit (Macherey-Nagel, Germany) according to the manufacturer's instructions. Finally, the 16S rRNA gene was sequenced at the sequencing service of the University of Salamanca (Nucleus-USAL).

### 1. Analysis of the functional (bioactive) properties of the selected strain

To analyze the functional (bioactive) properties of the isolated strain of the invention, fermentation of donkey milk was carried out both with the bacterium of the invention (strain denominated "strain 20", which corresponds to the strain *Lactococcus lactis* subsp. *lactis* CECT 31096), as well as with another bacterium belonging to the species *Lactococcus lactis* subsp. *lactis* (called "strain 12") and 3 others of the species *Lactococcus lactis* subsp. *hordniae* (called "strains 18, 28 and 31"), where the strains were grown in a liquid medium M17-L (with 1% lactose). The donkey milk was previously pasteurized at 74°C for 15 seconds (with a neutral pH, greater than or equal to 6.9). This fermentation was carried out, on the one hand, individually and, on the other hand, together with other starter cultures of yogurt or fermented milk.

The objective was to characterize the technological suitability of each of the bacterial strains. For this purpose, different parameters of the fermentation process were studied, such as: fermentation time, pH evolution, their effect on milk coagulation, the compatibility of each strain with commercial yogurt or fermented milk starter cultures, the number of bacteria at the end of fermentation and the sensory properties of the resulting product.

When comparing the behavior of the different bacterial strains during the fermentation of pasteurized donkey milk inoculated at 2%, it was seen that strains 18 and 20 took 4 hours to coagulate the milk and lower the pH to around 4.6. However, strain 12 took too long to grow (more than 4 hours), strain 28 took too little time (being able to intervene in the fermentation process) and strain 31 did not even grow under these conditions. In addition, it was observed that when the milk inoculated with strains 12 and 18 was stored under refrigeration, it showed a higher level of draining compared to the rest of the strains analyzed. On the other hand, when analyzing the organoleptic properties of the milk inoculated with the 5 selected strains, it was observed that the samples inoculated with strains 18 and 20 had better attributes (better aroma and flavour) compared to the rest of the samples.

In summary, the results obtained on the organoleptic and functional properties of the donkey milk inoculated with each of the five Lactococcus isolates under study showed that strain 20 had better qualities for fermenting donkey milk than strains 12, 18, 28 and 31.

### 2. Genetic sequencing of the selected strains

After extracting DNA from the 5 selected lactococcal strains (strains 12, 18, 20, 28 and 31) with the commercial NucleoSpin Microbial DNA Mini kit for DNA from microorganisms (Macherey-Nagel, Germany) according to the manufacturer's instructions and sequencing using Oxford Nanopore DNA sequencing technology, the genomes were assembled obtaining the complete genome and closed plasmids of all strains. We compared and analyzed using the TORMES program *(*Quijada et al., 2019, TORMES: an automated pipeline for whole bacterial genome analysis. Bioinformatics, 35(21), 4207-4212*);* *https:*//*github.com*/*nmquijada*/*tormes)* the genome of each of the 5 strains of Lactococcus (strains 12, 18, 20, 28 and 31), observing that strain 20 (*Lactococcus lactis* subsp. *lactis*) not only does not present genes for the production of biogenic amines, such as cadaverine, spermidine, spermine, phenylethylamine, histamine, putrescine, tyramine and tryptamine, but also contains candidate genes for resistance to bacteriophages (genes coding for a DNA methylase very similar to those of type LlaDII, which are related to phage resistance in *Lactococcus cremoris*), as well as genes encoding for proteins related to structural development, DNA/RNA repair, immunity (ACM lysozyme) and carbohydrate metabolism (alpha-amylase). In addition, it was found that this strain does not contain virulence genes and that it possesses in its chromosome the *ImrD* gene that codes for a protein that is an intrinsic toxicant extrusion pump of *L. lactis,* but that has been associated with resistance to lincosamide. Therefore, strain 20 is safe for health in terms of antibiotic resistance and virulence (QPS strain, qualified presumption of safety according to EFSA). In addition, strain 20 has the temperature resistance gene *CIpL* and genes related to the production of bacteriocins lactococcin A and sactipeptides (*BmbF*).

On the one hand, the fact that this strain contains genes related to the production of bacteriocin lactococcin A means that it can be considered an antimicrobial and biopreservative agent, as well as that it can be used as a biopreservative for various types of food. On the other hand, the fact that this strain contains the ClpL temperature resistance gene would demonstrate its potential use for the manufacture of all those products that require heat during the production process, such as cheese *(*Fowler. & Gasson, 1991, "Antibiotics-nisin". In Food preservatives. Russel, N.J. and Gould, G.W (eds.). pp. 135-152. Blackie*).*

### 3. Analysis of the antioxidant properties

The analysis of the antioxidant properties of each of the fractions (complete matrix, peptide fraction and phenolic fraction) of donkey milk fermented with strain 20, by measuring the antioxidant capacity to reduce iron or FRAP, which is used as a marker of antioxidant power, revealed interesting results (Figure 1). It was seen that the antioxidant capacity (reducing power index), obtained for the different fractions of the freeze-dried milk fermented with that strain (strain 20) was higher than that of the rest (strains 12, 18, 28 and 31), which would indicate that the use of that strain for fermentation would be able to increase the reducing power and, therefore, the antioxidant properties of the milk compared to the rest of the strains.

These results, together with those obtained after the analysis of the functional properties and possible industrial applications (sections 1 and 2), demonstrated the potential of strain 20, which is the reason we proceeded to its growth and subsequent lyophilization.

### 4. Bacterial growth and lyophilization

Cells of strain 20 were stored deep-frozen at -80°C in vials with glycerol for cryopreservation. In order to grow the bacteria in their culture medium, the frozen cells were first reconstituted. For this purpose, they were first incubated in their optimal medium (M17 broth with lactose) at 30°C for 16-20 hours without shaking. Once the cells were revitalized, they were inoculated in an optimal growth medium for larger scale production (composed of: whey, tryptone, yeast extract and distilled water) previously sterilized at 95°C for half an hour and pH adjusted to 7. Incubation was carried out at 30°C, without shaking and lasting 20-24 hours, until the pH of the medium was around 4.6 and the bacterial plate count in solid M17 culture medium was 1x10⁸ CFU/mL.

This liquid growth medium was deep-frozen and freeze-dried. The viability of the bacteria is adequate, since the number of colonies on plate after lyophilization remained in the order of 10⁸ CFU/g. The lyophilisate was stored in a protective nitrogen atmosphere for preservation.

### 5. Addition of the strain L. lactis CECT 31096 in combination with other commercial starter cultures to obtain a fermented product based on donkey milk

Once the strain of the invention (*Lactococcus lactis* subsp. *lactis* CECT 31096) was selected, and after its growth and lyophilization, it was added together with other commercial lactic acid bacteria (LAB) (*Streptococcus thermophilus* and *Lactobacillus bulgaricus* that constitute the commercial culture YF-L903 of CHR HANSEN) to a sample of pasteurized donkey milk. Afterwards, the fermentation process was carried out. To obtain the desired product, donkey milk pasteurized at 74°C for 15 seconds was used, the corresponding strains were inoculated and the fermentation process was carried out at a temperature of 45°C and a time of 3 hours, at which time the pH of the milk reached a value of 4.6.

The amount of *Lactococcus lactis* subsp. *lactis* used was 1 g of lyophilized product per 10 L of pasteurized donkey milk, while the amount of *Streptococcus thermophilus* and *Lactobacillus bulgaricus* added was established according to the manufacturer's specifications.

The manufacture of this product was based on the following objectives:
- Facilitate the digestion of lactose present in donkey milk by incorporating strains of the species *Streptococcus thermophilus* and *Lactobacillus bulgaricus* in its composition (these bacteria possess a B-galactosidase enzyme equivalent to that present in the human intestine, thus favoring lactose hydrolysis and metabolism at the gastrointestinal level).
- Provide greater microbiological diversity to donkey milk by incorporating strains of *Streptococcus thermophilus, Lactobacillus bulgaricus* and *Lactococcus lactis* subsp. *lactis* CECT 31096 in its composition.
- Improve the performance of the fermentation process and the organoleptic properties of donkey milk by incorporating *Streptococcus thermophilus, Lactobacillus bulgaricus* and *Lactococcus lactis* subsp. *lactis* CECT 31096 strains in its composition.

As a result of the fermentation process, a milk product with desirable organoleptic properties was obtained with a bacterial content (after plate counts in specific culture medium) of: 10⁸ CFU/mL of *Streptococcus thermophilus,* 10⁶ CFU/mL of *Lactobacillus bulgaricus* and 10⁸ CFU/mL of *Lactococcus lactis* subsp. *lactis* CECT 31096. As shown by the results of the count performed, the product obtained after fermentation of pasteurized donkey milk has a sufficient quantity of live microorganisms (10⁸) to be considered a probiotic.

### 6. Analysis of antioxidant properties of pasteurized and fermented donkey milk samples

In order to analyze the variations that the pasteurization and fermentation processes could induce in the antioxidant properties of donkey milk, the content of total phenols (TPs) and the antioxidant capacity markers ABTS and FRAP were measured in three different samples (Figure 2): pasteurized donkey milk (samples 240149 and 240150), pasteurized donkey milk fermented with yogurt starters (commercial culture YF-L903 from CHR HANSEN, samples 240151 and 240152) and pasteurized donkey milk fermented with the combination of yogurt starters (commercial culture YF-L903 from CHR HANSEN) together with the strain *Lactococcus lactis* subsp. *lactis* CECT 31096 (samples 240153 and 240154).

The analysis of the antioxidant markers (TPs, ABTS, FRAP) showed that the antioxidant properties of the pasteurized milk were lower than those of the milk fermented with both the commercial culture (YF-L903 from CHR HANSEN) and the mixed culture composed of the strain *Lactococcus lactis* subsp. *lactis* CECT 31096 and the commercial culture (YF-L903 from CHR HANSEN). These results demonstrate that the bacteria added to carry out the fermentation of donkey milk would be causing the hydrolysis of the proteins present in the same and with it, a greater bioavailability of bioactive peptides, which would translate into an increase of the antioxidant properties. Furthermore, when comparing the values obtained for fermented milk samples containing only the commercial culture (YF-L903 from CHR HANSEN) with those containing the mixed culture composed of *Lactococcus lactis* subsp. *lactis* CECT 31096 and the commercial culture (YF-L903 from CHR HANSEN), no significant differences in antioxidant properties were observed.

Therefore, it could be concluded that the strain *Lactococcus lactis* subsp. *lactis* CECT 31096 helps to preserve the antioxidant properties of the fermented product, acting in concert with the other LAB inoculated in the fermentation.

### 7. Analysis of antioxidant properties of products from digestion of fermented donkey milk samples

To complete the analysis of antioxidant properties (Figure 3), the content of total phenols (TPs) and the ABTS and FRAP markers of 2 digested samples were measured by means of a gastrointestinal simulator: pasteurized donkey milk fermented with yoghurt starters (CHR HANSEN YF-L903 sample 240155) and pasteurized donkey milk fermented with the combination of yoghurt starters (CHR HANSEN YF-L903) together with the strain *Lactococcus lactis* subsp. *lactis* CECT 31096 (sample 240156). The samples were digested by introducing 2.4724 g of fermented donkey milk powder dissolved in 25 mL of water in a dynamic gastrointestinal simulator of the ITACyL (Instituto Tecnológico Agrario de Castilla y León).

After analyzing the results obtained, it was observed that the content of total phenols (TPs) of the sample 240156 was significantly higher than that observed for sample 240155. This would indicate that the combination of the commercial yogurt cultures together with the *Lactococcus lactis* subsp. *lactis* strain CECT 31096 increases the phenol content after digestion from a value of 350 µmol GAE/100 g to 1170 µmol GAE/100 g. Furthermore, the same was seen to be true for the reducing power or FRAP, since the values obtained for sample 240156 were significantly higher than those of sample 240155. This would again indicate that the combination of the 3 strains mentioned above increases the reducing power after digestion, reaching a value of 1000 µmol reduced FE/100 g compared to the 750 µmol reduced FE/100 g obtained only in fermentation with commercial cultures without *Lactococcus lactis* subsp. *lactis* CECT 31096. For the ABTS marker, very similar values were obtained between both samples, close in both cases to 20000 µmol Eq. Trolox/100 g, although slightly higher in sample 240156.

Therefore, it could be concluded that the inoculation of the strain *Lactococcus lactis* subsp. *lactis* CECT 31096 during the fermentation of donkey milk with commercial yogurt cultures (YF-L903 from CHR HANSEN) is able to improve the antioxidant properties of the product obtained in comparison with that obtained by fermentation carried out only with these commercial cultures.

### 8. Analysis of the antihypertensive properties of pasteurized and fermented donkey milk samples

The antihypertensive properties were evaluated by the ability to inhibit angiotensin-converting enzyme (ACE). The inhibitory concentration was evaluated by IC₅₀. With different samples of both fermented and non-fermented donkey milk, it was observed that: to reduce ACE levels, an enzyme involved in hypertensive processes, at 50%, a much higher concentration of pasteurized milk (IC₅₀ of 7.23) would be required than that of milk fermented only with the commercial culture (YF-L903 from CHR HANSEN) (IC₅₀ of 3.58) and even more than that of milk fermented with the mixed culture composed of the strain *Lactococcus lactis* subsp. *lactis* CECT 31096 and the commercial culture (YF-L903 from CHR HANSEN) (IC₅₀ of 3.17). Based on these results, it is shown that a lower amount of milk fermented with the mixed culture would have the same antihypertensive effect as higher amounts of milk fermented only with the commercial culture (YF-L903 from CHR HANSEN) and even higher amounts of pasteurized milk. Therefore, it is concluded that the milk fermented with the mixed culture would have a greater antihypertensive effect compared to the rest of the milks analyzed (Figure 4).

When analyzing the IC₅₀ values obtained after ACE assays performed with different digested samples, by means of a gastrointestinal simulator, of freeze-dried donkey milk, it was observed that: to decrease the ACE enzyme activity (enzyme involved in hypertension) to 50%, a higher concentration of pasteurized donkey milk (IC₅₀ of 1.32) would be required than that of fermented only with the commercial culture (YF-L903 from CHR HANSEN) or fermented with the mixed culture composed of the strain *Lactococcus lactis* subsp. *lactis* CECT 31096 and the commercial culture (YF-L903 from CHR HANSEN) (IC₅₀ of 1.25). In this case, no differences were observed between the digests of milk fermented with the commercial culture YF-L903 of CHR HANSEN and milk fermented with the mixed culture. Based on these results it can be concluded that a lower amount of milk fermented with the mixed culture or only with the commercial culture (CHR HANSEN YF-L903) would have the same antihypertensive effect as higher amounts of pasteurized milk alone. Therefore, milk fermented with both the commercial culture (YF-L903 from CHR HANSEN) and the mixed culture composed of *Lactococcus lactis* subsp. *lactis* CECT 31096 and the commercial culture (YF-L903 from CHR HANSEN) would have a greater antihypertensive effect compared with the other milks tested (Figure 4).

It should be noted that the IC₅₀ values obtained for the digested milk samples were much lower than those of the undigested samples, which would indicate that the antihypertensive properties of donkey milk are greatly enhanced after ingestion.

These results reinforce the probiotic potential of the *Lactococcus lactis* subsp. *lactis* CECT 31096 strain and demonstrate that the fermented product manufactured from a mixed culture composed of *Lactococcus lactis* subsp. *lactis* CECT 31096 in combination with yogurt bacteria (*Streptococcus thermophilus* and *Lactobacillus bulgaricus* present in CHR HANSEN's YF-L903) has probiotic potential, since in addition to presenting a sufficient concentration of living microorganisms in the final product, it can generate a potential beneficial effect on the host's health (antioxidant and antihypertensive properties).

## Claims

1. A strain of *Lactococcus lactis* subsp. *lactis* with deposit number CECT 31096.

2. A strain derived from the strain according to claim 1.

3. A strain according to claim 1 or 2, wherein said strain is in the form of viable cells or in the form of non-viable cells.

4. A cellular component, metabolite, secreted molecule, or any combination thereof, obtained from the strain according to any one of claims 1 to 3.

5. A composition comprising a strain according to any one of claims 1 to 3, or a cellular component, metabolite, secreted molecule or any combination thereof according to claim 4.

6. A composition according to claim 5, wherein the composition additionally comprises at least one microorganism other than the strain according to any one of claims 1 to 3, preferably wherein the microorganism is *Streptococcus thermophilus* and/or *Lactobacillus delbrueckii* subsp. *bulgaricus.*

7. Composition according to claim 5 or 6, wherein said composition is a pharmaceutical composition.

8. Composition according to claim 7, wherein the composition additionally comprises at least one vehicle and/or a pharmaceutically acceptable excipient.

9. Composition according to claim 5 or 6, wherein said composition is a nutritional composition.

10. Composition according to claim 9, wherein the nutritional composition is a food, a supplement, a nutraceutical, a probiotic, a symbiotic or a postbiotic.

11. Composition according to claim 10, wherein the food is selected from the list consisting of a dairy product or an infant food, preferably a fermented dairy product from donkey milk.

12. Composition according to any one of claims 5 to 11, wherein said composition has a strain concentration of between 10⁴ and 10¹⁴ colony forming units (CFU) per gram or milliliter of final composition, preferably 10⁸ CFU/mL.

13. Non-therapeutic use of the strain according to any one of claims 1 to 3, a cellular component, metabolite, secreted molecule or any combination thereof according to claim 4, or a composition according to any one of claims 5, 6 or 9 to 12, for making a food, preferably a fermented dairy food from donkey milk.

14. Strain according to any one of claims 1 to 3, a cellular component, metabolite, secreted molecule or any combination thereof according to claim 4, or a composition according to any one of claims 5 to 12, for its use as a medicament.

15. Strain according to any one of claims 1 to 3, a cellular component, metabolite, secreted molecule or any combination thereof according to claim 4, or a composition according to any one of claims 5 to 12, for its use in the prevention and/or reduction of hypertension, or related conditions.
